(19) Europäisches Patentamt
European Patent Office
Office européen des brevets

(11) Veröffentlichungsnummer: **0 368 212 B1**

(12) **EUROPÄISCHE PATENTSCHRIFT**

(45) Veröffentlichungstag der Patentschrift: **20.09.95**

(51) Int. Cl.$^{6}$: **A01N 25/32**

(21) Anmeldenummer: **89120520.5**

(22) Anmeldetag: **06.11.89**

(54) **Herbizide Mittel, die 2-(4-Heteroaryloxy)- oder 2-(4-Aryloxy)-phenoxyessig- oder -propionsäurederivate und/oder Cyclohexenonderivate als herbizide Wirkstoffe und Naphthalinderivate als Antidots enthalten, sowie ihre Verwendung zur Bekämpfung unerwünschten Pflanzenwuchses.**

(30) Priorität: **09.11.88 DE 3837926**

(43) Veröffentlichungstag der Anmeldung:
**16.05.90 Patentblatt 90/20**

(45) Bekanntmachung des Hinweises auf die Patenterteilung:
**20.09.95 Patentblatt 95/38**

(84) Benannte Vertragsstaaten:
**CH DE FR GB IT LI NL**

(56) Entgegenhaltungen:
**EP-A- 0 012 717**
**CA-A- 1 202 498**
**DE-A- 1 952 910**
**DE-A- 3 029 645**
**DE-A- 3 808 355**
**US-A- 3 749 566**

**WEED SCIENCE Bd. 32, Nr. 1, Januar 1984, GAINESVILLE,FLA, US; Seiten 51 - 58; K.K.HATZIOS: 'Interactions Between Selected Herbicides and Protectants on Corn (Zea mays)'**

(73) Patentinhaber: **BASF Aktiengesellschaft**
**Carl-Bosch-Strasse 38**
**D-67063 Ludwigshafen (DE)**

(72) Erfinder: **Hagen, Helmut, Dr.**
**Max-Slevogt-Strasse 17 e**
**D-6710 Frankenthal (DE)**
Erfinder: **Pfister, Juergen, Dr.**
**Ziegelofenweg 1**
**D-6720 Speyer (DE)**
Erfinder: **Eichenauer, Ulrich, Dr.**
**Paul-Ehrlich-Strasse 25 a**
**D-6000 Frankturt 70 (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**
Erfinder: **Helbig, Wilfried, Dr.**
**Johann-Gottlieb-Fichte-Strasse 67**
**D-6730 Neustadt (DE)**

**EP 0 368 212 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

| | |
|---|---|
| **ZEITSCHRIFT FüR NATURFORSCHUNG , C: BIOSCIENCES Bd. 46C, Nr. 9/10, 1991, TüBINGEN, DE; Seiten 934 - 938; K.K.HATZIOS: 'Effects of the Herbicide Sethoxydim and the Safener Dichlorimid on Lipid Synthesis and Acetyl-CoA Carboxylase Activity of Grain Sorghum'** | |

## Beschreibung

Die Vorliegende Erfindung betrifft herbizide Mittel, die 2-(4-Heteroaryloxy)- oder 2-(4-Aryloxy)-phenoxyessig- oder -propionsäurederivate und/oder Cyclohexenonderivate als herbizide Wirkstoffe und Naphthalinderivate als Antidots enthalten, sowie Verfahren zur selektiven Bekämpfung von unerwünschtem Pflanzenwuchs mit diesen herbiziden Mitteln.

Herbizide Wirkstoffe aus der Gruppe der 2-(4-Heteroaryloxy)- oder 2-(4-Aryloxy)-phenoxyessigsäurederivate der Formel II

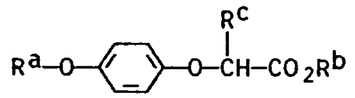

II

in der die Substituenten folgende Bedeutung haben:

$R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, $C_1$- bis $C_4$-Alkyl, $C_1$- bis $C_4$-Halogenalkyl und/oder Nitro;

$R^b$ Wasserstoff, eine $C_1$- $C_4$-Alkylgruppe oder das Äquivalent eines pflanzenverträglichen Kations und

$R^c$ Wasserstoff oder eine Methylgruppe

oder aus der Gruppe der Cyclohexenonderivate der Formel III

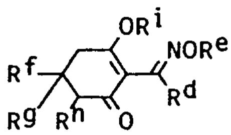

III

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche einfach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff- oder Schwefelatom enthalten kann, wobei das Schwefelatom bis zu zwei Sauerstoffatome tragen kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
ein 10-gliedriger gesättigter oder einfach ungesättigter Heterocyclus, welcher zwei Sauerstoff- oder Schwefelatome enthält und durch bis zu drei $C_1$-$C_4$-Alkylgruppen und/oder Methoxygruppen substituiert sein kann;
ein Phenylrest, ein Pyridylrest oder ein Isoxazolylrest, wobei dieser Ring bis zu drei der folgenden Gruppen tragen können:
$C_1$-$C_4$-Alkyl, $C_1$-$C_4$-Halogenalkyl, $C_1$-$C_4$-Alkoxy, $C_1$-$C_4$-Alkylthio,
$C_3$-$C_6$-Alkenyloxy, $C_3$-$C_6$-Alkinyloxy, $C_1$-$C_4$-Alkoxy-$C_1$-$C_3$-alkyl,
$C_1$-$C_4$-Dialkoxy-$C_1$-$C_3$-alkyl, Formyl, Halogen und/oder Benzoylamino

$R^g$ Wasserstoff oder eine $C_1$-$C_6$-Alkylgruppe;

$R^h$ Wasserstoff, eine Cyanogruppe, ein Halogenatom oder eine $C_1$-$C_4$-Alkoxycarbonylgruppe und

$R^i$ Wasserstoff oder das Äquivalent eines umweltverträglichen Kations

sind aus der Literatur bekannt.

2-(4-Heteroaryloxy)- oder 2-(4-Aryloxy)-phenoxyessigsäurederivate (z.B. DE-A 22 23 894, DE-A 24 33 067, DE-A 30 04 770, BE-A 868 875 und BE-A 858 618) dienen der Bekämpfung von unerwünschten Pflanzen aus der Familie der Gramineen. Die Verträglichkeit dieser Substanzen für Kulturpflanzen variiert jedoch zwischen kommerziell acceptabel und unverträglich, je nach Substituenten und Aufwandmenge.

Cyclohexenonderivate sind in der Literatur (z.B. EP-A 228 598, EP-A 230 235, EP-A 238 021, US-A 4 432 786, DE-A 24 39 104) ebenfalls als Herbizide beschrieben. Sie dienen vorwiegend zur Bekämpfung unerwünschter Gräser in dicotylen Kulturen. Je nach Struktur der Substituenten und Dosis der Anwendung können Verbindungen aus dieser Gruppe auch zur selektiven Bekämpfung von unerwünschten Gräsern in Gramineen Kulturen wie Weizen und Reis eingesetzt werden.

Naphthalinderivate sind in der Literatur sowohl als Herbizide als auch als Antidot der Formel I' beschrieben,

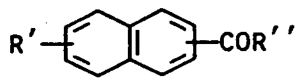

I'

in der die Substituenten u.a. folgende Bedeutung haben:

R' Wasserstoff, Halogen, Nitro, $C_1$-$C_5$-Alkyl oder Phenyl und

R" Hydroxy, Alkoxy, Alkylthio, Amino oder substituiertes Amino

(HU-A 21 318, HU-A 21 319, HU-A 21 320, HU-A 21 321, HU-A 21 322, HU-A 21 323 und BE 884 634). Diese Antidots werden für die Verminderung von Schäden empfohlen, die durch Herbizide der folgenden Klassen hervorgerufen werden: Harnstoffe, Thiocarbamate, Triazine, Chloracetanilid- und Urazilderivate.

In Weed Science 32(1), 51-58 (1984) wird diskutiert, inwieweit vier verschiedene Antidots die Kulturpflanze Mais vor der herbiziden Wirkung von u.a. 2-[4-[5-(Trifluormethyl-2-pyridyl)oxy]phenoxy]-propionsäurebutylester und 2-[1-(Ethoxyimino)-butyl]-5-[2-(ethylthio)-propyl]-3-hydroxy-2-cyclohexen-1-on schützen können. Das zu den vorliegenden Naphthalinderivaten I strukturell ähnlichste Antidot 1,8-Naphthalindicarbonsäureanhydrid zeigte nur bei Vorauflauf-applikation einen gewissen antagonistischen Effekt auf die genannten Herbizide.

Aus der DE-A 19 52 910 ist bekannt, das Getreidesamen durch Behandlung mit 1,8-Naphthalindicarbonsäure, deren niederen Alkylestern, Bariumsalzen, Zinnsalzen oder Monoamiden vor der phytotoxischen Wirkung von N,N-Dialkylthiocarbamaten geschützt werden können.

Schließlich wird in der U.S. 3,749,566 gelehrt, daß Reissamen durch die Behandlung mit einem Aminsalz der 1,8-Naphthalincarbonsäure, insbesondere mit dem Dimethylammonium- oder Diethanolammoniumsalz, gegen die herbizide Wirkung von N,N-Dialkylthiocarbamat-Estern resistent gemacht werden können.

Aufgabe der vorliegenden Erfindung war es, Verbindungen zu finden, welche in der Lage sind, die Schäden an Kulturpflanzen zu kompensieren, welche durch Herbizide des (Heteroaryloxy)- bzw. (Aryloxy)-phenoxyessigsäure- bzw. Cyclohexenontyps hervorgerufen werden.

Entsprechend der Aufgabe wurde gefunden, daß sich Naphthalinderivate der Formel I

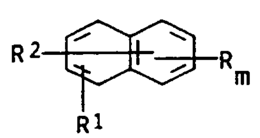

I

in der die Substituenten und der Index folgende Bedeutung haben:

R $C_1$-$C_4$-Alkyl, $C_1$-$C_4$-Alkylthio, $C_1$-$C_4$-Halogenalkyl, $C_1$-$C_4$-Alkoxy, Halogen, Hydroxy, Nitro oder Benzyl;

m 0, 1, 2 oder 3, wobei die Reste R verschieden sein können, wenn m 2 oder 3 bedeutet;

$R^1$ -CN; -C(NH$_2$)NOH;

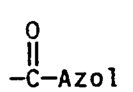 , 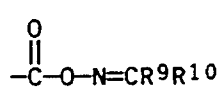 oder 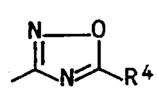 ;

$R^2$ Halogen; -NR$^5$R$^6$; -N=CH-R$^5$; -NR$^5$-SO$_2$R$^7$; COOR$^3$; OH

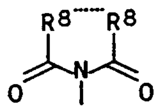 oder 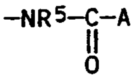

und

$R^3$ Wasserstoff,
$C_1$-$C_6$-Alkyl, welches ein bis drei der folgenden Substituenten tragen kann: Halogen, $C_1$-$C_4$-Alkylamino, Di-($C_1$-$C_4$-Alkyl)amino, $C_1$-$C_4$-Alkoxy und $C_1$-$C_4$-Alkylthio;
Phenyl oder Benzyl, welche ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Cyano, Nitro und/oder Halogen;

$R^4$ Phenyl, welches ein oder zwei der folgenden Gruppen tragen kann: Halogen, $C_1$-$C_4$-Alkyl, $C_1$-$C_4$-Alkoxy, $C_1$-$C_4$-Alkylthio, $C_1$-$C_4$-Halogenalkyl, Cyano und/oder Nitro;

$R^5$ Wasserstoff;
$C_1$-$C_6$-Alkyl, welches ein bis drei der folgenden Substituenten tragen kann: Halogen, $C_1$-$C_4$-Alkylamino, Di-($C_1$-$C_4$-Alkyl)amino, $C_1$-$C_4$-Alkoxy, $C_1$-$C_4$-Alkylthio, Phenyl und/oder ($C_1$-$C_4$-Alkyl)oxycarbonyl;
Phenyl, welches ein bis drei der folgenden Substituenten tragen kann: Halogen, $C_1$-$C_4$-Alkyl, $C_1$-$C_4$-Alkoxy, $C_1$-$C_4$-Alkylthio, $C_1$-$C_4$-Halogenalkyl, Cyano und/oder Nitro;

$R^6$ Wasserstoff;
$C_1$-$C_6$-Alkyl, welches ein bis drei der folgenden Substituenten tragen kann: Halogen, $C_1$-$C_4$-Alkylamino, Di-($C_1$-$C_4$-Alkyl)amino, $C_1$-$C_4$-Alkoxy, $C_1$-$C_4$-Alkylthio und/oder Phenyl;

$R^7$ $C_1$-$C_6$-Alkyl, welches ein bis drei der folgenden Substituenten tragen kann: $C_1$- $C_4$-Alkoxy, $C_1$- $C_4$-Alkylthio, $C_1$-$C_4$-Alkylamino, Di-($C_1$-$C_4$-Alkyl)amino, Phenyl und/oder ein bis fünf Halogenatome;
Phenyl oder 5- bis 6-gliedriges Heteroaryl, wobei diese Ringe ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Nitro, Cyano und/oder Halogen;

$R^8$ $C_1$-$C_6$-Alkyl oder $C_2$-$C_6$-Alkenyl, wobei diese Gruppen ein bis drei der folgenden Substituenten tragen können: Halogen, $C_1$-$C_4$-Alkoxy, $C_1$-$C_4$-Alkylthio und/oder Phenyl;
Phenyl, welches ein bis drei der folgenden Substituenten tragen kann: $C_1$-$C_4$-Alkyl, $C_1$-$C_4$-Alkoxy, $C_1$-$C_4$-Alkylthio, $C_1$-$C_4$-Halogenalkyl, Nitro, Cyano und/oder Halogen;
oder $R^8$...$R^8$ gemeinsam
eine $C_2$-$C_4$-Alkylen- oder $C_2$-$C_4$-Alkenylenkette bilden, wobei diese Brücken bis zu drei der folgenden Substituenten tragen können: $C_1$-$C_4$-Alkyl, $C_1$-$C_4$-Alkoxy, $C_1$-$C_4$-Alkylthio, Halogen und/oder Phenyl, oder
einen anellierten Benzolring bilden, welcher ein bis vier Halogenatome und/oder ein bis drei der folgenden Reste tragen kann: $C_1$-$C_4$-Alkyl, $C_1$-$C_4$-Alkoxy, $C_1$-$C_4$-Alkylthio, $C_1$-$C_4$-Halogenalkyl, Cyano, Nitro und/oder Phenyl;

$R^9$, $R^{10}$ $C_1$-$C_4$-Alkyl oder gemeinsam mit dem C-Atom, an das sie gebunden sind $C_3$-$C_6$-Cycloalkyl;

A Wasserstoff,
$C_1$-$C_6$-Alkyl, welches ein bis drei der folgenden Substituenten tragen kann: Halogen, $C_1$-$C_4$-Alkoxy, $C_1$-$C_4$-Alkylthio, $C_1$-$C_4$-Alkylamino, Di-($C_1$-$C_4$-Alkyl)amino, Phenyl und/oder $C_1$-$C_4$-Alkoxycarbonyl;
$C_1$-$C_4$-Alkoxycarbonyl;
-$NR^5R^6$- oder
Phenyl, welches ein bis fünf Halogenatome und/oder ein bis drei der folgenden Substituenten tragen kann: $C_1$-$C_4$-Alkyl, $C_1$-$C_4$-Alkoxy, $C_1$-$C_4$-Halogenalkyl, Cyano und/oder Nitro;

ausgezeichnet zum Schutz von Kulturpflanzen aus der Familie der Gramineen eignen und speziell in Kulturen wie Getreide, Reis, Mais und Sorghum die Verträglichkeit der Herbizide des (Heteroaryloxy)- oder Aryloxyphenoxyessigsäure- bzw. Cyclohexenon-Typs verbessern. Die Schädigungen an den Nutzpflanzen werden abgeschwächt oder ganz unterbunden. Unerwünschte Grasarten werden bekämpft, wobei es für die Wirkung gleichgültig ist, ob diese herbiziden Mittel, bestehend aus herbizidem Wirkstoff und antidotischer Verbindung gemeinsam oder getrennt formuliert und ausgebracht werden bzw. bei getrennter Ausbringung, in welcher Reihenfolge herbizider Wirkstoff und Antidot appliziert werden.

Die Herstellung der Verbindungen I erfolgt nach in der Literatur allgemein üblichen Methoden.

Derivate I, in denen die Reste $R^1$ und $R^2$ benachbarte Ringpositionen einnehmen, erhält man beispielsweise, indem man ein entsprechendes Nitronaphthylderivat IV analog zu den von Y. Tomioka (Chem. Pharm. Bull. 33 (4), 1360-1366 (1985)) beschriebenen Bedingungen zum Cyanonaphthylamin Ia ($R^1$ = CN, $R^2$ = $NH_2$) umsetzt. Diese Reaktionsfolge ist für die Synthese von 2-Aminonaphthalin-1-carbonitrilderivaten im folgenden Schema dargestellt:

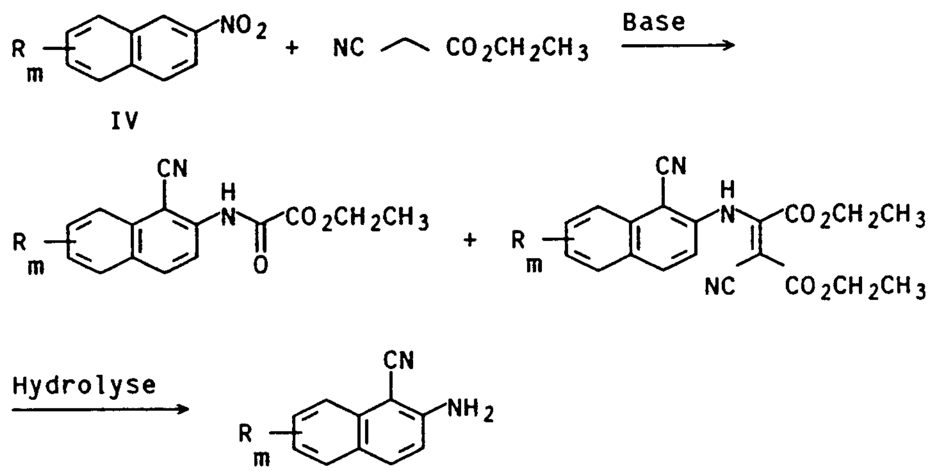


Aus den Derivaten Ia lassen sich die entsprechenden Verbindungen I mit Hilfe der literaturüblichen Derivatisierungsmethoden synthetisieren (Derivate der Cyanogruppe: Methoden der organ. Chem. (Houben-Weyl) Band E5, S. 263 ff, S. 710 ff, S. 790 ff, S. 941 ff, S. 1242 ff, S. 1313; Band X/4 (1968), S. 209 ff; Derivate der Aminogruppe: Methoden der organ. Chem. (Houben-Weyl) Band XI (2), S. 3 ff, Band V (2) S. 846).

Die Synthese der Naphthalinderivate I, in denen $R^1$ und $R^2$ nicht benachbarte Ringpositionen einnehmen, ist auf vielfältige Weise möglich und in der Literatur beschrieben. Die Literatur-Zitate sind mit den entsprechenden Verbindungen in Tabelle 1 bzw. 2 im Anschluß an die Synthesebeispiele aufgeführt.

Im Hinblick auf die biologische Wirksamkeit sind Naphthalinderivate bevorzugt, in denen die Substituenten folgende Bedeutung haben:

R Alkyl wie Methyl, Ethyl, Propyl, 1-Methylethyl, Butyl, 1-Methylpropyl, 2-Methylpropyl und 1,1-Dimethylethyl, insbesondere Methyl und Ethyl;
Halogenalkyl wie Fluormethyl, Difluormethyl, Trifluormethyl, 2,2-Difluorethyl, 2,2,2-Trifluorethyl und 1,2,2-Trifluorethyl, insbesondere Trifluormethyl und 2,2,2-Trifluorethyl;
Alkoxy wie Methoxy, Ethoxy, Propyloxy, iso-Propyloxy und ter.-Butyloxy, insbesondere Methoxy und Ethoxy;
Alkylthio wie Methylthio, Ethylthio, iso-Propylthio und tert.-Butylthio, insbesondere Methylthio;
Halogen wie Fluor, Chlor, Brom und Jod, insbesondere Fluor, Chlor und Brom; Hydroxyl; Nitro oder Benzyl;

m 0, 1, 2 oder 3, wobei die Reste R verschieden sein können, wenn m 2 oder 3 bedeutet;

$R^1$ Cyano; ein Carboxaminoxim, Oxadiazol, eine Iminoestergruppe, worin $R^9$ und $R^{10}$ für Methyl, Ethyl, Propyl, Butyl, Cyclopentyl oder Cyclohexyl stehen oder ein Rest -C(O)-Azol, worin Azol einen 1-Imidazolyl-, 1-Pyrazolyl- und insbesondere 1-Triazolylrest bedeutet.

Besonders bevorzugt sind:

- Cyano,
- 3-Oxadiazolylreste, welche in 5-Position durch einen Phenylring substituiert sind, wobei dieser Phenylrest wiederum bis zu drei Substituenten tragen kann. Als Substituenten kommen hier die bereits unter R genannten Halogenatome, Alkyl-, Alkoxy-, Alkylthio-, Halogenalkyl- und/oder Cyano- bzw. Nitrogruppen in Betracht.

$R^2$ kann die Bedeutung Halogenatom wie unter R genannt besitzen, wobei Fluor, Chlor und Brom bevorzugt sind.
Daneben kann $R^2$ auch eine Carbonsäuregruppe, eine Hydroxylgruppe, eine Aminogruppe oder ein Derivat einer Hydroxygruppe oder einer Aminogruppe bedeuten. Besonders bevorzugt sind hierbei

- Dialkylaminogruppen wie Dimethylamino und Diethylamino

- Diacylaminogruppen wie Diacetylamino, Maleinimino, Dichlormaleinimino, Methylmaleinimino, Phthalimino, Tetrabromophthalimino und 3,4,5,6-Tetrahydrophthalimino,
- Acylaminogruppen wie Acetylamino, Glutarylamino, Benzoylamino und Ethyloxaloylamino
- Sulfonylamingruppen wie Phenylsulfonylamino und N-(Phenylsulfonyl)-N-(1-carboxylyl)-methylamino
- Iminogruppen wie Benzalimino.

Als Derivate von Hydroxygruppen kommen Salze wie Alkalimetallsalze oder Ester mit anorganischen oder organischen Säuren wie Alkylsulfonsäuren, Phosphorsäuren oder Carbonsäuren in Betracht.

Von den Verbindungen I sind solche ganz besonders bevorzugt, in denen die Reste $R^1$ und $R^2$ benachbarte Ringpositionen einnehmen.

Spezielle Beispiele für herbizide (Heteroaryloxy)- bzw. Aryloxy-phenoxyessigsäurederivate der Formel II, deren Kulturpflanzenverträglichkeit durch Naphthalinderivate der Formel I verbessert werden kann, sind in der folgenden Tabelle A aufgeführt:

Tabelle A

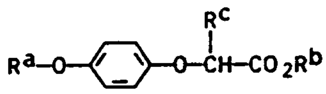

II

| Nr. | $R^a$ | $R^b$ | $R^c$ | Lit |
|---|---|---|---|---|
| II.1 | 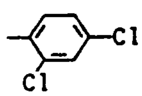 | $CH_3$ | $CH_3$ | DE-A 22 23 894 |
| II.2 | 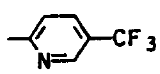 | $n-C_4H_9$ | $CH_3$ | BE-A 868 875 |
| II.3 | 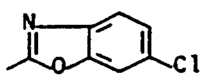 | $C_2H_5$ | $CH_3$ | BE-A 858 618 |
| II.4 | 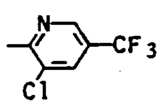 | $CH_3$ | $CH_3$ | BE-A 868 875 |
| II.5 | 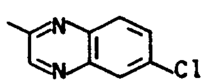 | $C_2H_5$ | $CH_3$ | DE-A 30 04 770 |

Spezielle Beispiele für Cyclohexenone der Formel III, deren Kulturpflanzenverträglichkeit durch Naphthalinderviate der Formel I verbessert werden kann, sind in der folgenden Tabelle B aufgeführt.

Tabelle B

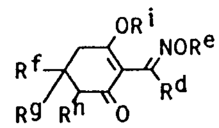 III

| Nr. | $R^d$ | $R^e$ | $R^f$ | $R^g$ | $R^h$ | $R^i$ | Literatur |
|---|---|---|---|---|---|---|---|
| III.1 | $C_3H_7$ | $CH_2CH{=}CH_2$ | $CH_3$ | $CH_3$ | $CO_2CH_3$ | Na | DE-A 2 439 104 |
| III.2 | $C_3H_7$ | $CH_2CH_3$ | $CH_2CH(CH_3)SCH_2CH_3$ | H | H | H | DE-A 2 822 304 |
| III.3 | $C_2H_5$ | $CH_2CH{=}CHCl$ | $CH_2CH(CH_3)SCH_2CH_3$ | H | H | H | US-A 4 440 566 |
| III.4 | $C_3H_7$ | $CH_2CH{=}CHCl$ | $CH_2CH(CH_3)SCH_2CH_3$ | H | H | H | US-A 4 440 566 |
| III.5 | $C_3H_7$ | $C_2H_5$ | 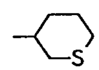 | H | H | H | EP-A 71 707 |
| III.6 | $C_2H_5$ | $C_2H_5$ | 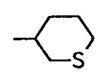 | H | H | H | EP-A 71 707 |

| Nr. | $R^d$ | $R^e$ | $R^f$ | $R^g$ | $R^h$ | $R^i$ | Literatur |
|---|---|---|---|---|---|---|---|
| III.7 | $CH_3$ | $CH_2CH=CHCH_3$ | 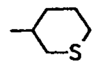 | H | H | H | EP-A 71 707 |
| III.8 | $C_3H_7$ | $C_2H_5$ | 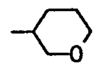 | H | H | H | EP-A 71 707 |
| III.9 | $C_2H_5$ | $CH_2CH=CHCl$ | 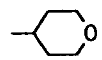 | H | H | H | EP-A 142 741 |
| III.10 | $C_3H_7$ | $C_2H_5$ | 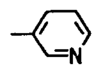 | H | H | H | EP-A 66 195 |
| III.11 | $C_2H_5$ | $C_2H_5$ | 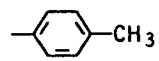 | H | H | H | DE-A 24 39 104 |
| III.12 | $C_2H_5$ | $CH_2CH=CHCH_3$ | 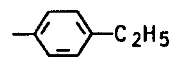 | H | H | H | DE-A 38 08 072 |
| III.13 | $C_2H_5$ | $C_2H_5$ | 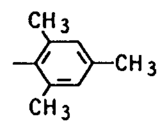 | H | H | H | EP-A 880 301 |
| III.14 | $C_3H_7$ | $CH_2CH=CHCl$ | 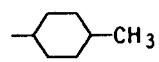 | H | H | H | EP-A 88 299 |
| III.15 | $C_3H_7$ | $CH_2CH=CHCH_3$ | 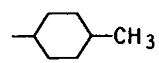 | H | H | H | EP-A 88 299 |

| Nr. | $R^d$ | $R^e$ | $R^f$ | $R^g$ | $R^h$ | $R^i$ | Literatur |
|---|---|---|---|---|---|---|---|
| III.16 | $C_2H_5$ | $CH_2CH{=}CHCH_3$ | 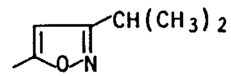 | H | H | H | EP-A 238 021 |
| III.17 | $C_3H_7$ | $CH_2CH{=}CHCH_3$ | 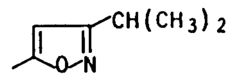 | H | H | H | EP-A 238 021 |
| III.18 | $C_2H_5$ | $CH_2CH{=}CHCl$ | 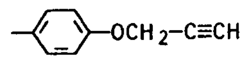 | H | H | H | EP-A 137 174 |
| III.19 | $C_3H_7$ | $C_2H_5$ | 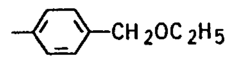 | H | H | H | EP-A 2 137 200 |
| III.20 | $C_3H_7$ | $C_2H_5$ | 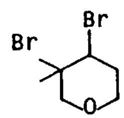 | H | H | H | EP-A 230 235 |
| III.21 | $C_3H_7$ | $CH_2CH{=}CHCl$ | 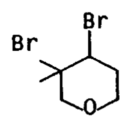 | H | H | H | EP-A 230 235 |
| III.22 | $C_2H_5$ | $C_2H_5$ | 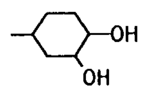 | H | H | H | EP-A 278 598 |

| Nr. | $R^d$ | $R^e$ | $R^f$ | $R^g$ | $R^h$ | $R^i$ | Literatur |
|---|---|---|---|---|---|---|---|
| III.23 | $C_3H_7$ | $CH_2CH=CHCl$ | 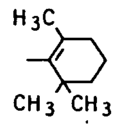 | H | H | H | EP-A 46 860 |
| III.24 | $C_3H_7$ | $C_2H_5$ | 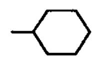 | H | H | H | JP-A 540 191 945 |
| III.25 | $C_3H_7$ | $C_2H_5$ | 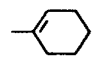 | H | H | H | EP-A 46 860 |
| III.26 | $CH_3$ | $CH_2CH=CHCl$ | 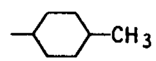 | H | H | H | EP-A 88 299 |
| III.27 | $C_3H_7$ | $C_2H_5$ | 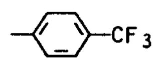 | H | H | K | EP-A 137 174 |
| III.28 | $C_2H_5$ | $CH_2CH=CHCl$ | 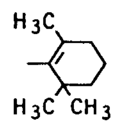 | H | H | H | EP-A 46 860 |

Synthesebeispiele

Beispiel 1

2-Benzoylaminonaphthalin-1-carbonitril

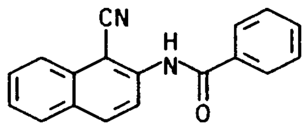

3,0 g (17,8 mmol) 2-Aminonaphthalin-1-carbonitril (Chem. Pharm. Bull. 33 (4), 1360-1366 (1985)) wurden mit 2,5 g (18,1 mmol) Benzoylchlorid und 4 ml Triethylamin in 100 ml Toluol 15 Stunden bei 80 °C gerührt. Nach beendeter Reaktion wurde das Produkt abfiltriert und getrocknet. Man erhielt so 1,5 g (31 % d. Th.) der Titelverbindung vom Fp. 205 °C. (Wirkstoffbeispiel 1.008)

Beispiel 2

1-(N-Phthalimido)naphthalin-2-carbonitril

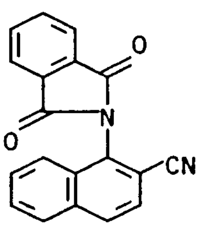

3,0 g (17,9 mmol) 1-Aminonaphthalin-2-carbonitril (Chem. Pharm. Bull. 33 (4), 1360-1366 (1985)) und 2,7 g (17,9 mmol) Phthalsäureanhydrid wurden 15 Stunden in 40 ml Essigsäure (konz.) zum Sieden erhitzt. Nach beendeter Umsetzung wurde die Reaktionsmischung in 200 ml Wasser eingerührt, der Niederschlag isoliert und getrocknet. Man erhielt so 3,5 g (66 % d. Th.) der Titelverbindung vom Fp. 196 °C (Wirkstoffbeispiel 2.010).

Beispiel 3

5-(3-chlorphenyl)-3-(1'-aminonapht-2'-yl)-oxadiazol

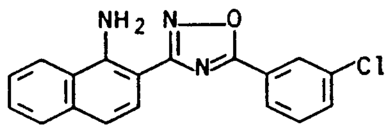

a) 1-Aminonaphthalin-2-carbonsäureamidoxim

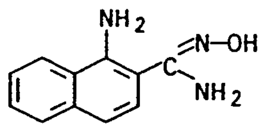

33,6 g (0,20 mol) 1-Aminonaphthalin-2-carbonitril (Chem. Pharm. Bull. 33, 1360-1366 (1985)), 18,8 g (0,27 mol) Hydroxylammoniumchlorid und 22,7 g (0,27 mol) Natriumhydrogencarbonat wurden 15 Stunden in 300 ml Ethano/Wasser (3:2) unter Rückfluß erhitzt. Nach dem Abkühlen saugte man ab und wusch mit n-Propanol nach. Ausbeute 30,0 g (75 %) Titelverbindung vom Fp. = 178-180 °C (Wirkstoffbeispiel 2.031).

b) 6,0 g (30 mmol) der unter a) erhaltenen Verbindung, 6,9 ml (50 mmol) 3-Chlorbenzoesäuremethylester und 3,2 g (60 mmol) Natriummethylat wurden in 70 ml Methanol 15 Stunden zum Sieden erhitzt. Nach beendeter Umsetzung wurde in 500 ml Wasser eingerührt, das ausgefallene Produkt abfiltriert und getrocknet. Man erhielt so 5,4 g (56 % d. Th.) der Titelverbindung vom Fp. = 160 °C (Wirkstoffbeispiel 2.069).

Beispiel 4

2-Chlornaphthalin-1-carbonitril

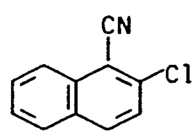

33,6 g (0,2 mol) 2-Aminonaphthalin-1-carbonitril (s. Beispiel 1) in 250 ml (abs.) Acetonitril wurden bei 65 °C zu einer Suspension von 32,3 g (0,24 mol) Kupfer(II)chlorid (wasserfrei) und 30,9 g (0,3 mol) t-Butylnitril in 800 ml (abs.) Acetonitril gegeben. Nach beendeter Gasentwicklung wurde die abgekühlte Reaktionsmischung mit 2000 ml 20%iger Salzsäure versetzt, der Niederschlag isoliert, gewaschen und getrocknet. Man erhielt so 31,7 g (70 % d. Th.) der Titelverbindung vom Fp. = 82-83 °C. (Wirkstoffbeispiel 1.001)

Tabelle 1

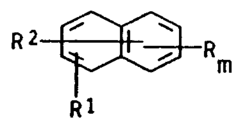

| Nr. | $R^1$ | $R^2$ | $R_m$ | Fp [°C] | Literatur |
|---|---|---|---|---|---|
| 1.001 | 1-CN | 2-Cl | | 98 - 99 | Chem. Ber. 83, 171 (1950) |
| 1.002 | 1-CN | 2-Br | | 85 - 86 | |
| 1.003 | 1-CN | $2-NH_2$ | | 122 | Chem. Pharm. Bull 33, 1360 (1985) |
| 1.004 | 1-CN | $2-NH_2$ | $6-OCH_3$ | | J. Org. Chem. 33, 1719 (1968) |
| 1.005 | 1-CN | $2-NHCOCH_3$ | | 164 | Monatsh. Chem. 89, 358 (1958) |
| 1.006 | 1-CN | $2-N(COCH_3)_2$ | | 111-113 | wie 1.005 |
| 1.007 | 1-CN | $2-NHCO(CH_2)_2CO_2H$ | | 204 | |
| 1.008 | 1-CN | 2-NHCOPh | | 205 | |
| 1.009 | 1-CN | 2-NHCONHPh | | 270 | |
| 1.010 | 1-CN | $2-NHCOCO_2CH_2CH_3$ | | 162 | wie 1.003 |
| 1.011 | 1-CN | 2-N=CHPh | | 112-113 | wie 1.005 |
| 1.012 | 1-CN | 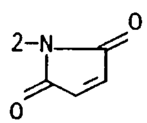 | | 148 | |
| 1.013 | 1-CN | 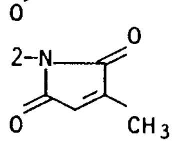 | | 96 | |

Tabelle 1 - Fortsetzung -

| Nr. | $R^1$ | $R^2$ | $R_m$ | Fp [°C] | Literatur |
|---|---|---|---|---|---|
| 1.014 | 1-CN | 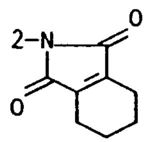 | | 83 | |
| 1.015 | 1-CN | 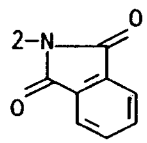 | | 139 | |
| 1.016 | 1-CN | 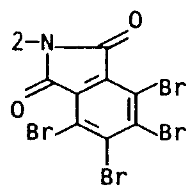 | | >260 | |
| 1.017 | 1-CN | $3-NH_2$ | | 116-118 | Bull. Soc. Chim. Fr. 234 (1954) |
| 1.018 | 1-CN | $3-NHCOCH_3$ | | 260 | wie 1.017 |
| 1.019 | 1-CN | 4-Cl | | 110 | Chem. Ber. 28, 1840 |
| 1.020 | 1-CN | $4-NH_2$ | | 174 | Ber. Dts. Chem. Ges. 28, 1842 |
| 1.021 | 1-CN | $4-NH_2$ | $2-CH_3$ | 188 | J. Am. Chem. Soc. 78, 4774 (1956) |
| 1.022 | 1-CN | $4-NH_2$ | $3-NO_2$ | 239,5 | CA 3353 (1956) |
| 1.023 | 1-CN | $4-NHCOCH_3$ | | 189,5 | wie 1.022 |
| 1.024 | 1-CN | $4-NHCOCH_3$ | $3-NO_2$ | 270,5 | wie 1.022 |
| 1.025 | 1-CN | $4-NHSO_2Ph$ | $3-CH_3$ | 183-184 | J. Am. Chem. Soc. 79, 170 (1957) |
| 1.026 | 1-CN | $4-NHSO_2Ph$<br>$CH_2CO_2H$ | $3-CH_3$ | 195,5-196,5 | wie 1.025 |

Tabelle 1 - Fortsetzung -

| Nr. | $R^1$ | $R^2$ | $R_m$ | Fp [°C] | Literatur |
|---|---|---|---|---|---|
| 1.027 | 1-CN | 5-Cl | | 148 | J. Chem. Soc. P.T.I. 2779 (1979) |
| 1.028 | 1-CN | 5-Br | | 147 | Chem. Ber. 9, 1516 |
| 1.029 | 1-CN | $5-NH_2$ | | 139-140 | a) J. Chem. Soc. 678 (1954)979) |
| | | | | | b) J. Am. Chem. Soc. 63, 828 (1941) |
| 1.030 | 1-CN | $5-NHCOCH_3$ | | | |
| 1.031 | 1-CN | 5-NHCOPh | | 211-212 | Helv. Chim. Acta 8, 8419 (1979) |
| 1.032 | 1-CN | 6-Cl | | | |
| 1.033 | 1-CN | 6-Br | | | |
| 1.034 | 1-CN | $6-NH_2$ | | | |
| 1.035 | 1-CN | 7-Cl | | | |
| 1.036 | 1-CN | 7-Br | | | |
| 1.037 | 1-CN | $7-NH_2$ | | | |
| 1.038 | 1-CN | 8-Cl | | 145 | wie 1.027 |
| 1.039 | 1-CN | 8-Br | | | |
| 1.040 | 1-CN | $8-NH_2$ | | 88 | Bull. Soc. Chim. Fr. 1561 (1972) |

Tabelle 1 - Fortsetzung -

| Nr. | $R^1$ | $R^2$ | $R_m$ | Fp [°C] | Literatur |
|---|---|---|---|---|---|
| 1.041 | 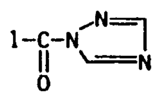 | H | 2,7-$Cl_2$ | | |
| 1.042 | 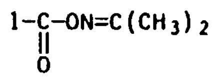 | H | 2,7-$Cl_2$ | | |
| 1.043 | 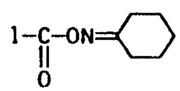 | H | 2,7-$Cl_2$ | 155-158 | |

Tabelle 1 - Fortsetzung -

| Nr. | $R^1$ | $R^2$ | $R_m$ | Fp [°C] | Literatur |
|---|---|---|---|---|---|
| 1.044 | 1-CN | 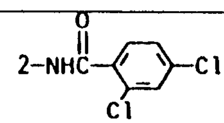 | | 134-136 | |
| 1.045 | 1-CN | 2-OH | | | |

Tabelle 2

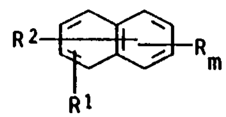

| Nr. | $R^1$ | $R^2$ | $R_m$ | Fp [°C] | Literatur |
|---|---|---|---|---|---|
| 2.001 | 2-CN | 1-Cl | | 101-102 | |
| 2.002 | 2-CN | 1-Br | | 85-86 | |
| 2.003 | 2-CN | 1-Br | 6-Br | 178 | J. Chem. Soc. 1426 (1958) |
| 2.004 | 2-CN | $1-NH_2$ | | 109 | Bull.Soc.Chim.Fr. 1561 (1972); Chem.Pharm.Bull.33, 1360 (1985) |
| 2.005 | 2-CN | $1-NH_2$ | $3-CH_2Ph$ | | |
| 2.006 | 2-CN | $1-NHCOCH_3$ | | 219.5-220.5 | Rec. Trav. Chim. Pays-Bas 76, 401 (1957) |
| 2.007 | 2-CN | 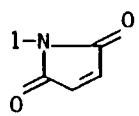 | | >260 | |
| 2.008 | 2-CN | 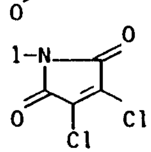 | | 222 | |
| 2.009 | 2-CN | 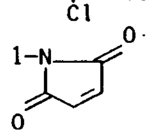 | | 186 | |
| 2.010 | 2-CN | 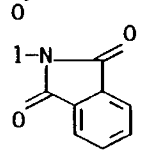 | | 196 | |

Tabelle 2 - Fortsetzung -

| Nr. | $R^1$ | $R^2$ | $R_m$ | Fp [°C] | Literatur |
|---|---|---|---|---|---|
| 2.011 | 2-CN | 3-$NH_2$ | | 163-164 | Bull. Soc. Chim. Fr. 743 (1954) |
| 2.012 | 2-CN | 3-$NH_2$ | 4-Br | 154-155 | J.Prakt.Chem. 319, 65 (1977) |
| 2.013 | 2-CN | 3-Cl | | | |
| 2.014 | 2-CN | 3-Br | | | |
| 2.015 | 2-CN | 4-Cl | | | |
| 2.016 | 2-CN | 4-Br | | | |
| 2.017 | 2-CN | 4-$NH_2$ | | 125-126 | wie 1.029b) |
| 2.018 | 2-CN | 5-Cl | | 142<br>144 | wie 1.027<br>J. Prakt. Chem. 43, 412 |
| 2.019 | 2-CN | 5-Cl | 8-Cl | 140 | J.Chem.Soc. 1426 (1958) |
| 2.020 | 2-CN | 5-$NH_2$ | | 143-144 | wie 1.029b) |
| 2.021 | 2-CN | 6-Cl | | | |
| 2.022 | 2-CN | 6-$NH_2$ | | 199 | wie 1.029b) |
| 2.023 | 2-CN | 7-Cl | | | |
| 2.024 | 2-CN | 7-Br | | | |
| 2.025 | 2-CN | 7-$NH_2$ | | 197 | wie 1.029a) |
| 2.026 | 2-CN | 8-Cl | | 112-113 | wie 1.027 |
| 2.027 | 2-CN | 8-Br | | | |
| 2.028 | 2-CN | 8-$NH_2$ | | 133 | DRP 92 995 |
| 2.029 | 2-C($NH_2$)=NOH | 1-Cl | | | |

Tabelle 2 - Fortsetzung -

| Nr. | $R^1$ | $R^2$ | $R_m$ | Fp [°C] | Literatur |
|---|---|---|---|---|---|
| 2.030 | 2-C($NH_2$)=NOH | 1-Br | | | |
| 2.031 | 2-C($NH_2$)=NOH | 1-$NH_2$ | | 164-166 | |

Tabelle 2 - Fortsetzung -

| Nr. | R1 | R2 | $R_m$ | Fp [°C] | Literatur |
|---|---|---|---|---|---|
| 2.032 | 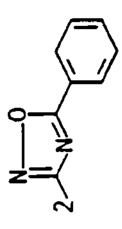 | 1-$NH_2$ | | 132-134 | |
| 2.033 | 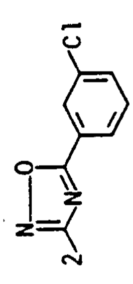 | 1-$NH_2$ | | 160 | |
| 2.034 | 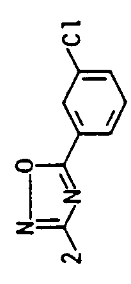 | 1-Cl | | 160 | |
| 2.035 | 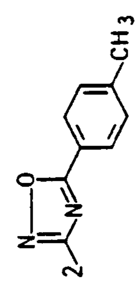 | 1-Cl | | | |
| 2.036 | 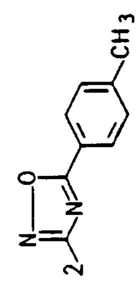 | 1-$NH_2$ | | 141-142 | |

Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen

Beispiele aus Gewächshausversuchen belegt:

Als Kulturgefäße dienen Plastikblumentöpfe mit rund 300 $cm^3$ Inhalt und lehmiger Sand mit etwa 3,0 % Humus als Substrat. Die Samen der Testpflanzen werden nach Arten getrennt, flach eingesät und befeuchtet. Danach deckt man die Gefäße mit durchsichtigen Plastikhauben ab, bis die Samen gleichmäßig gekeimt und die Pflanzen angewachsen sind.

Für die Nachauflaufbehandlung zieht man die Testpflanzen je nach Wuchsform erst bis zu einer Wuchshöhe von 3 bis 20 cm an und behandelt sie dann. Die herbiziden Mittel werden hierbei in Wasser als Verteilungsmittel suspendiert oder emulgiert und mittels fein verteilender Düsen gespritzt.

Als Beispiel für 2-(4-Heteroaryloxy)-phenoxysäurederivate der Formel II dient II.3:

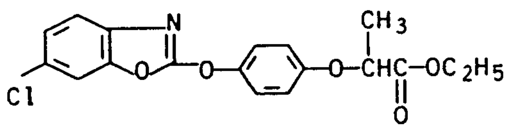

II.3

In den biologischen Beispielen aufgeführte Cyclohexenonderivate III sind:

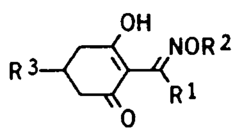

III

| No. | $R^3$ | $R^1$ | $R^2$ |
|---|---|---|---|
| III.2 | 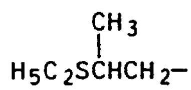 | $C_3H_7$ | $C_2H_5$ |
| III.12 | 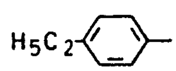 | $C_2H_5$ | $-CH_2-CH=CH-CH_3$ |
| III.16 | 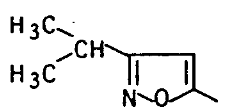 | $C_2H_5$ | $-CH_2CH=CHCH_3$ |
| III.13 | 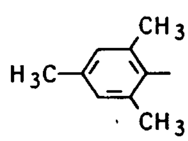 | $C_2H_5$ | $C_2H_5$ |
| III.5 | 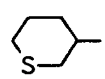 | $C_3H_7$ | $C_2H_5$ |

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Denkbar ist auch eine Behandlung der Kulturpflanzensamen mit dem Antidot vor der Aussaat. Der herbizide Wirkstoff wird dann allein in der üblichen Weise appliziert.

Für herbizide (Heteroaryloxy)phenoxyessigsäurederivate II werden unterschiedliche Mengen einer antidotisch wirkenden Verbindung benötigt, wenn das Herbizid in verschiedenen Kulturen eingesetzt wird. Die Mengenverhältnisse sind in breiten Bereichen variabel. Sie sind ebenfalls abhängig von der Struktur der (Heteroaryloxy)phenoxyessigsäurederivate II und der jeweiligen Zielkultur. Geeignete Anteilverhältnisse herbizider Wirkstoffe: antidotisch wirkender Verbindung liegen zwischen 1 : 40 bis 1 : 0,01; vorzugsweise ebenfalls zwischen 1 : 4 bis 1 : 0,1 Gew.-Teile.

Für das gleiche Cyclohexenonderivat III werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat III und ein Naphthalinderivat I eingesetzt werden, sind in breiten Bereichen variabel. Sie sind abhängig von der Struktur des Cyclohexenonderivats, des Naphthalinderivats und der jeweiligen Kultur. Geeignete Anteilverhältnisse herbizider Wirkstoff: antidotisch wirkender Verbindung liegen zwischen 1 : 40 bis 1 : 0,01; vorzugsweise 1 : 4 bis 1 : 0,25 Gew.-Teile.

Die neuen herbiziden Mittel können neben dem Naphthalinderivat I als Antidot und dem Herbizid aus der Gruppe der (Heteroaryloxy)phenoxyessigsäuren II bzw. der Cyclohexenone III weitere herbizide oder wachstumsregulierende Wirkstoffe anderer chemischer Struktur enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.-% an herbizidem Wirkstoff und Antidot, vorzugsweise zwischen 0,5 und 90 Gew.-%. Die Aufwandmengen an herbizidem Wirkstoff betragen 0,2 bis 5 kg/ha.

Der herbizide Wirkstoff III.2 wird als kommerziell formuliertes Produkt (184 g/l Emulsionskonzentrat (EC)) auch allein jeweils unter Zugabe von derjenigen Menge an Lösungsmittelsystem in die Spritzbrühe eingesetzt, mit welcher die antidotisch wirkende Verbindung in den Tabellen angegebenen Aufwandmengen ausgebracht werden.

Bei den anderen herbiziden Wirkstoffen unterließ man diesen Zusatz, wenn das Herbizid nicht mit einer antidotisch wirkenden Verbindung kombiniert wurde.

Sie waren wie folgt aufbereitet:
III.12, III.16 und III.13 als 100 g/l EC; II.3 als 120 g/l EC und III.5 als 200 g/l EC.

Sämtliche antidotisch wirkende Verbindungen werden in einem Gemisch, bestehend aus 80 % Lösungsmittel und 20 % Tensid, mit 10 % Gew.-% Wirkstoff aufbereitet.

Die Versuchsgefäße werden im Gewächshaus aufgestellt, wobei für wärmeliebende Arten 18 bis 35° und für solche gemäßigtere Klimate 10 bis 25° bevorzugt werden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt. Bewertet wurde die Schädigung durch die chemischen Mittel anhand einer Skala von 0 % bis 100 % im Vergleich zu den unbehandelten Kontrollpflanzen. Dabei bedeutet 0 keine Schädigung und 100 eine völlige Zerstörung der Pflanzen.

| Liste der Testpflanzen: | | |
|---|---|---|
| Lat. Name | Deutscher Name | Englischer Name |
| Alopecurus myosuroides | Ackerfuchsschwarz | blackgrass |
| Avena fatua | Flughafer | wild oats |
| Hordeum vulgare | Gerste | barley |
| Lolium multiflorum | ital. Raygras | annual raygrass |
| Oryza sativa | Reis | rice |
| Setaria faberii | Borstenhirse | giant foxtail |
| Setaria italica | Kolbenhirse | foxtail millet |
| Setaria viridis | Grüne Borstenhirse | green foxtail |
| Triticum aestivum | Weizen | wheat |
| Zea mays | Mais | Indian corn |

Die anschließenden Tabellen dokumentieren die antidotische Wirkung, wobei die Beispielsverbindungen Nr. 1.001, 1.002, 1.003, 1.045 und 2.004, die Kulturpflanzenverträglichkeit der Cyclohexenonderivate III.2, III.5, III.12, III.16, III.13 und III.22 und des (Heteroaryloxy)phenoxysäurederivats II.3

Tabelle 3

| Verbesserte Verträglichkeit des Herbizids Sethoxydim III.2 bei Weizen durch Zumischung antidotischer Verbindungen und Nachauflaufwendung im Gewächshaus | | | | |
|---|---|---|---|---|
| Antidot Nr. | Aufwandmenge kg/ha | | Testpflanzen und Schädigung % | |
| | III.2 | Antidot | Kulturpflanze Weizen * | unerwünschte Pflanze Lolium multifl. |
| - | 0,015 | - | 50 | 100 |
| | 0,03 | - | 65 | 100 |
| 1.003 | 0,015 + 0,06 | | 10 | 100 |
| | 0,03 + 0,125 | | 25 | 100 |
| 1.002 | 0,015 + 0,06 | | 10 | 100 |
| | 0,03 + 0,125 | | 25 | 100 |

*Sorte "Okapi"

Tabelle 4

| Antidot Nr. | Aufwandmenge kg/ha | | Testpflanzen und Schädigung % | |
|---|---|---|---|---|
| | III.2 | Antidot | Kulturpflanze Weizen ** | unerwünschte Pflanze Setaria viridis |
| — | 0,0125<br>0,025 | -<br>- | 40<br>90 | 95<br>98 |
| 1.003 | 0,0125 + 0,025 | | 0 | 90 |
| | 0,025 + 0,05 | | 0 | 100 |

**Sorte "Okapi"

Tabelle 5

| Verbesserung der Verträglichkeit des Herbizids Sethoxydim III.2 gegenüber der Kulturpflanze Mais durch Zumischung antidotischer Verbindungen und Nachauflaufanwendung im Gewächshaus | | | | |
|---|---|---|---|---|
| Antidot Nr. | Aufwandmenge kg/ha | | Testpflanzen und Schädigung % | |
| | III.2 | Antidot | Kulturpflanze Mais ** | unerwünschte Pflanze Lolium multifl. |
| - | 0,015<br>0,03 | -<br>- | 65<br>80 | 100<br>100 |
| 2.004 | 0,015 + 0,06 | | 0 | 98 |
| | 0,03 + 0,125 | | 20 | 100 |
| 1.001 | 0,015 + 0,06 | | 30 | 100 |
| | 0,03 + 0,125 | | 20 | 100 |

**Sorte "Inrakorn"

Tabelle 6

| Verbesserung der Verträglichkeit von Sethoxydim III.2 für Reis durch Mischung mit der antidotisch wirkenden Verbindung Nr. 1.003 bei Nachauflaufanwendung im Gewächshaus | | | | |
|---|---|---|---|---|
| Antidot Nr. | Aufwandmenge kg/ha | | Testpflanzen und Schädigung % | |
| | III.2 | Antidot | Kulturpflanze Reis ** | unerwünschte Pflanze Setaria italica |
| 1.003 | 0,015 | - | 60 | 98 |
| | 0,015 + 0,125 | | 15 | 98 |

** Reissorte "Bahia"

Tabelle 7

| Verbesserung der Verträglichkeit verschiedener Herbizide für Weizen durch Kombinieren mit der antidotischen Verbindung Nr. 1.003 bei Nachauflaufanwendung im Gewächshaus | | | | |
|---|---|---|---|---|
| Herbizider Wirkstoff | Aufwandmenge kg/ha | | Testpflanzen und Schädigung % | |
| | III | Antidot | Kulturpflanze Weizen ** | unerwünschte Pflanze Avena fatua |
| III.12 | 0,125<br>0,25 | -<br>- | 40<br>50 | 100<br>100 |
| III.12 | 0,25 + 0,5 | | 0 | 95 |
| III.16 | 0,125<br>0,25 | -<br>- | 20<br>50 | 100<br>100 |
| III.16 | 0,125 + 0,5 | | 0 | 100 |
| | 0,25 + 0,5 | | 0 | 100 |
| III.13 | 0,06<br>0,125 | -<br>- | 20<br>50 | 100<br>100 |
| III.13 | 0,06 + 0,5 | | 0 | 98 |
| | 0,125 + 0,5 | | 0 | 95 |

**Sorte "Okapi"

Tabelle 8

| Verbesserung der Verträglichkeit des Herbizids Cycloxydim III.5 für Gerste durch Mischung mit der anti dotischen Verbindung Nr. 1.003 bei Nachauflaufanwendung im Gewächshaus | | | | |
|---|---|---|---|---|
| Aufwandmenge kg/ha | | Testpflanzen und Schädigung % | | |
| III.5 | Antidot | Kulturpflanze | unerwünschte Pflanze | |
| | | Gerste *** | Avena fatua | Setaria fab. |
| 0,015<br>0,03 | -<br>- | 30<br>98 | 100<br>100 | 98<br>100 |
| 0,015 + 0,5 | | 10 | 100 | 98 |
| 0,03 + 0,5 | | 20 | 100 | 100 |

*** Sorte "Igri"

Tabelle 9

| Verbesserung der Verträglichkeit von Fenoxaprop-ethyl II.3 für Getreide durch Mischung mit der antidotischen Verbindung Nr. 1.003 bei Nachauflaufanwendung im Gewächshaus | | | | |
|---|---|---|---|---|
| Aufwandmenge kg/ha | | Testpflanzen und Schädigung % | | |
| II.3 | Antidot | Kulturpflanze | | unerwünschte Pflanze |
| | | Gerste*** | Weizen** | Setaria fab. |
| 0,03 | - | 30 | 40 | 100 |
| 0,06 | - | 60 | 50 | 100 |
| 0,03 + 0,5 | | 10 | 0 | 100 |
| 0,06 + 0,5 | | 20 | 0 | 100 |

** Sorte "Okapi"
*** Sorte "Igri"

Tabelle 10

Verbesserung der Verträglichkeit von Sethoxydim III.2 für Weizen durch Vorbehandlung der Pflanzen mit der antidotisch wirkenden Verbindung 1.003 bei Nachauflaufanwendung im Gewächshaus 24h vor der Applikation von III.2

| Herbizid Nr. | Aufwandmenge kg/ha | | Testpflanzen und Schädigung % | |
|---|---|---|---|---|
| | III | Antidot | Kulturpflanze Weizen** | unerwünschte Pflanze Lolium multiflorum |
| III.2 | 0,03 | - | 30 | 100 |
| | 0,03 + 0,125 | | 0 | 100 |
| | 0,03 + 0,5 | | 0 | 100 |

** Sorte "Jubilar"

Tabelle 11

| Verbesserung der Verträglichkeit von III.22 für Weizen durch Zumischung der antidotisch wirkenden Verbindung 1.045 bei Nachauflaufanwendung im Gewächshaus | | | | |
|---|---|---|---|---|
| Herbizid Nr. | Aufwandmenge kg/ha | | Testpflanzen und Schädigung % | |
| | III | Antidot | Kulturpflanze Weizen** | unerwünschte Pflanze Lolium multiflorum |
| III.22 | 0,06 | - | 18 | 100 |
| | 0,125 | - | 62 | 100 |
| | 0,06 + 0,25 | | 0 | 100 |
| | 0,06 + 0,125 | | 0 | 100 |
| | 0,125 + 0,5 | | 12 | 100 |
| | 0,125 + 0,125 | | 18 | 100 |

Tabelle 12

| Verbesserung der Verträglichkeit von III.2 für Weizen durch Mischung mit der antidotischen Verbindung Nr. 1.045 bei Nachauflaufanwendung im Gewächshaus | | | |
|---|---|---|---|
| Aufwandmenge kg/ha | | Testpflanzen und Schädigung % | |
| III.2 | Antidot | Kulturpflanze Weizen** | unerwünschte Pflanze Alopecurus myosuroides |
| 0,03 | - | 30 | 100 |
| 0,06 | - | 62 | 100 |
| 0,03 | 0,03 | 5 | 100 |
| 0,03 | 0,06 | 0 | 100 |
| 0,03 + 0,125 | | 0 | 100 |
| 0,06 + 0,06 | | 18 | 98 |
| 0,06 + 0,25 | | 5 | 100 |

** Sorte "Okapi", "Kanzler"

**Patentansprüche**

**1.** Herbizide Mittel, gekennzeichnet durch einen Gehalt an mindestens einem Naphthalinderivat der Formel I,

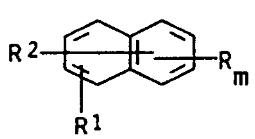

I

in der die Substituenten und der Index folgende Bedeutung haben:

R $C_1$-$C_4$-Alkyl, $C_1$-$C_4$-Alkylthio, $C_1$-$C_4$-Halogenalkyl, $C_1$-$C_4$-Alkoxy, Halogen, Hydroxy, Nitro oder Benzyl;

m 0, 1, 2 oder 3, wobei die Reste R verschieden sein können, wenn m 2 oder 3 bedeutet;

$R^1$ -CN; -$C(NH_2)NOH$;

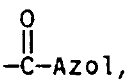, 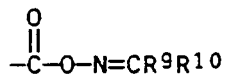 oder 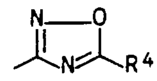 ;

$R^2$ Halogen; -$NR^5R^6$; -$N=CH-R^5$; -$NR^5-SO_2R^7$; $COOR^3$; OH

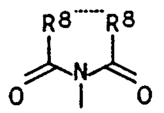 oder 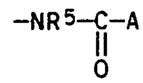

und

$R^3$ Wasserstoff,
$C_1$-$C_6$-Alkyl, welches ein bis drei der folgenden Substituenten tragen kann: Halogen, $C_1$-$C_4$-Alkylamino, Di-($C_1$-$C_4$-Alkyl)amino, $C_1$-$C_4$-Alkoxy und $C_1$-$C_4$-Alkylthio;
Phenyl oder Benzyl, welche ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Cyano, Nitro und/oder Halogen;

$R^4$ Phenyl, welches ein oder zwei der folgenden Gruppen tragen kann: Halogen, $C_1$-$C_4$-Alkyl, $C_1$-$C_4$-Alkoxy, $C_1$-$C_4$-Alkylthio, $C_1$-$C_4$-Halogenalkyl, Cyano und/oder Nitro;

$R^5$ Wasserstoff;
$C_1$-$C_6$-Alkyl, welches ein bis drei der folgenden Substituenten tragen kann: Halogen, $C_1$-$C_4$-Alkylamino, Di-($C_1$-$C_4$-Alkyl)amino, $C_1$-$C_4$-Alkoxy, $C_1$-$C_4$-Alkylthio, Phenyl und/oder ($C_1$-$C_4$-Alkyl)oxycarbonyl;
Phenyl, welches ein bis drei der folgenden Substituenten tragen kann: Halogen, $C_1$-$C_4$-Alkyl, $C_1$-$C_4$-Alkoxy, $C_1$-$C_4$-Alkylthio, $C_1$-$C_4$-Halogenalkyl, Cyano und/oder Nitro;

$R^6$ Wasserstoff;
$C_1$-$C_6$-Alkyl, welches ein bis drei der folgenden Substituenten tragen kann: Halogen, $C_1$-$C_4$-Alkylamino, Di-($C_1$-$C_4$-Alkyl)amino, $C_1$-$C_4$-Alkoxy, $C_1$-$C_4$-Alkylthio und/oder Phenyl;

$R^7$ $C_1$-$C_6$-Alkyl, welches ein bis drei der folgenden Substituenten tragen kann: $C_1$- $C_4$-Alkoxy, $C_1$- $C_4$-Alkylthio, $C_1$-$C_4$-Alkylamino, Di-($C_1$-$C_4$-Alkyl)amino, Phenyl und/oder ein bis fünf Halogenatome;
Phenyl oder 5- bis 6-gliedriges Heteroaryl, wobei diese Ringe ein bis drei der folgenden Gruppen tragen können: $C_1$-$C_4$-Alkyl, $C_1$-$C_4$-Alkoxy, $C_1$-$C_4$-Alkylthio, $C_1$-$C_4$-Halogenalkyl, Nitro, Cyano und/oder Halogen;

$R^8$ $C_1$-$C_6$-Alkyl oder $C_2$-$C_6$-Alkenyl, wobei diese Gruppen ein bis drei der folgenden Substituenten tragen können: Halogen, $C_1$-$C_4$-Alkoxy, $C_1$-$C_4$-Alkylthio und/oder Phenyl;
Phenyl, welches ein bis drei der folgenden Substituenten tragen kann: $C_1$-$C_4$-Alkyl, $C_1$-$C_4$-Alkoxy, $C_1$-$C_4$-Alkylthio, $C_1$-$C_4$-Halogenalkyl, Nitro, Cyano und/oder Halogen;
oder $R^8$...$R^8$ gemeinsam
eine $C_2$-$C_4$-Alkylen- oder $C_2$-$C_4$-Alkenylenkette bilden, wobei diese Brücken bis zu drei der folgenden Substituenten tragen können: $C_1$-$C_4$-Alkyl, $C_1$-$C_4$-Alkoxy, $C_1$-$C_4$-Alkylthio, Halogen und/oder Phenyl, oder
einen anellierten Benzolring bilden, welcher ein bis vier Halogenatome und/oder ein bis drei der folgenden Reste tragen kann: $C_1$-$C_4$-Alkyl, $C_1$-$C_4$-Alkoxy, $C_1$-$C_4$-Alkylthio, $C_1$-$C_4$-Halogenalkyl, Cyano, Nitro und/oder Phenyl;

$R^9$, $R^{10}$ $C_1$-$C_4$-Alkyl oder gemeinsam mit dem C-Atom, an das eingebunden sind $C_3$-$C_6$-Cycloalkyl;

A Wasserstoff,
$C_1$-$C_6$-Alkyl, welches ein bis drei der folgenden Substituenten tragen kann: Halogen, $C_1$-$C_4$-Alkoxy, $C_1$-$C_4$-Alkylthio, $C_1$-$C_4$-Alkylamino, Di-($C_1$-$C_4$-Alkyl)amino, Phenyl und/oder $C_1$-$C_4$-Alkoxycarbonyl;
$C_1$-$C_4$-Alkoxycarbonyl;
-$NR^5R^6$- oder

Phenyl, welches ein bis fünf Halogenatome und/oder ein bis drei der folgenden Substituenten tragen kann: $C_1$-$C_4$-Alkyl, $C_1$-$C_4$-Alkoxy, $C_1$-$C_4$-Halogenalkyl, Cyano und/oder Nitro;

als antagonistischem Mittel sowie mindestens einem herbiziden Wirkstoff aus der Gruppe

a) der 2-(4-Heteroaryloxy)- oder 2-(4-Aryloxy)-phenoxyessigsäurederivate der Formel II

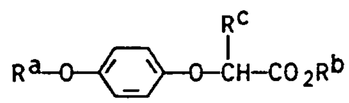 II

in der die Substituenten folgende Bedeutung haben:

$R^a$ ein Phenylring, ein Pyridylring, ein Benzoxazylrest, ein Benzthiazylrest oder ein Benzpyrazinylrest, wobei diese aromatischen Ringsysteme bis zu zwei der folgenden Reste tragen können: Halogen, $C_1$-$C_4$-Alkyl, $C_1$-$C_4$-Halogenalkyl und/oder Nitro;

$R^b$ Wasserstoff, eine $C_1$-$C_4$-Alkylgruppe oder das Äquivalent eines pflanzenverträglichen Kations und

$R^c$ Wasserstoff oder eine Methylgruppe und/oder,

b) der Cyclohexenonderivate der Formel III

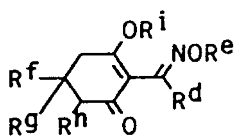 III

in welcher die Substituenten folgende Bedeutung haben:

$R^d$ eine $C_1$-$C_4$-Alkylgruppe;

$R^e$ eine $C_1$-$C_4$-Alkylgruppe, eine $C_3$-$C_4$-Alkenylgruppe, eine $C_3$-$C_4$-Alkinylgruppe, eine $C_3$-$C_4$-Halogenalkylengruppe oder eine Thenylgruppe, welche durch ein Halogenatom substituiert sein kann;

$R^f$ eine $C_1$-$C_4$-Alkylgruppe, welche einfach durch $C_1$-$C_4$-Alkylthio oder $C_1$-$C_4$-Alkoxy substituiert sein kann;
ein 5- oder 6-gliedriges gesättigtes oder einfach ungesättigtes Ringsystem, welches neben Kohlenstoffgliedern ein Sauerstoff- oder Schwefelatom enthalten kann, wobei das Schwefelatom bis zu zwei Sauerstoffatome tragen kann, und dieser Ring bis zu drei der folgenden Reste tragen kann: Hydroxy, Halogen, $C_1$-$C_4$-Alkyl, $C_1$-$C_4$-Halogenalkyl, $C_1$-$C_4$-Alkoxy und/oder $C_1$-$C_4$-Alkylthio;
ein 10-gliedriger gesättigter oder einfach ungesättigter Heterocyclus, welcher zwei Sauerstoffatome oder Schwefelatome enthält und durch bis zu drei $C_1$-$C_4$-Alkylgruppen und/oder Methoxygruppen substituiert sein kann;
ein Phenylrest, ein Pyridylrest oder ein Isoxazolylrest, wobei diese Reste bis zu drei der folgenden Gruppen tragen können: $C_1$-$C_4$-Alkyl, $C_1$-$C_4$-Halogenalkyl, $C_1$-$C_4$-Alkoxy, $C_1$-$C_4$-Alkylthio, $C_3$-$C_6$-Alkenyloxy, $C_3$-$C_6$-Alkinyloxy, $C_1$-$C_4$-Alkoxy-$C_1$-$C_3$-alkyl, $C_1$-$C_4$-Dialkoxy-$C_1$-$C_3$-alkyl, Formyl, Halogen und/oder Benzoylamino

$R^g$ Wasserstoff oder eine $C_1$-$C_6$-Alkylgruppe;

$R^h$ Wasserstoff, eine Cyanogruppe, ein Halogenatom oder eine $C_1$-$C_4$-Alkoxycarbonylgruppe und

$R^i$ Wasserstoff oder das Äquivalent eines umweltverträglichen Kations.

**2.** Herbizides Mittel nach Anspruch 1, dadurch gekennzeichnet, daß das Anteilsverhältnis Herbizid der Formel II/Naphthalinderivat bei gemeinsamer oder getrennter Ausbringung 1:4 bis 1:0,01 Gew.-Teile beträgt.

3. Herbizides Mittel nach Anspruch 1, dadurch gekennzeichnet, daß das Anteilsverhältnis Herbizid der Formel III/Naphthalinderivat bei gemeinsamer oder getrennter Ausbringung 1:4 bis 1:0,01 Gew.-Teile beträgt.

4. Verfahren zur selektiven Bekämpfung von unerwünschtem Pflanzenwuchs, dadurch gekennzeichnet, daß man ein Naphthalinderivat der Formel I gemäß Anspruch 1 und ein 2-(4-Heteroaryloxy)- oder 2-(4-Aryloxy)-phenoxyessigsäurederivat der Formel II vor, bei oder nach der Aussaat der Kulturpflanzen, vor oder während des Auflaufens der Kulturpflanzen gleichzeitig oder nacheinander ausbringt.

5. Verfahren zur selektiven Bekämpfung von unerwünschtem Pflanzenwuchs, dadurch gekennzeichnet, daß man ein Naphthalinderivat der Formel I gemäß Anspruch 1 und ein Cyclohexenonderivat der Formel III vor, bei oder nach der Aussaat der Kulturpflanzen, vor oder während des Auflaufens der Kulturpflanzen gleichzeitig oder nacheinander in beliebiger Reihenfolge ausbringt.

6. Verfahren zur Verhinderung von Schädigungen von Kulturpflanzen durch herbizide 2-(4-Heteroaryloxy)- oder 2-(4-Aryloxy)-phenoxyessigsäurederivate der Formel II gemäß Anspruch 1, dadurch gekennzeichnet, daß man das Saatgut der Kulturpflanzen mit einer antagonistisch wirksamen Menge eines Naphthalinderivats der Formel I gemäß Anspruch 1 behandelt.

7. Verfahren zur Verhinderung von Schädigungen von Kulturpflanzen durch herbizide Cyclohexenonderivate der Formel III gemäß Anspruch 1, dadurch gekennzeichnet, daß man das Saatgut der Kulturpflanzen mit einer antagonistisch wirksamen Menge eines Naphthalinderivats der Formel I gemäß Anspruch 1 behandelt.

8. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem Naphthalinderivat der Formel I gemäß Anspruch 1 und mit einem 2-(4-Heteroaryloxy)- oder 2-(4-Aryloxy)-phenoxyessigsäurederivat der Formel II gleichzeitig oder nacheinander behandelt.

9. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem Naphthalinderivat der Formel I gemäß Anspruch 1 und mit einem Cyclohexenonderivat der Formel III gleichzeitig oder nacheinander behandelt.

10. Verfahren gemäß den Ansprüchen 4 bis 9, dadurch gekennzeichnet, daß die Kulturpflanzen Gerste, Weizen, Mais, Kultursorghum und Reis sind.

**Claims**

1. A herbicide containing, as antagonistic agent, at least one naphthalene derivative of the formula I

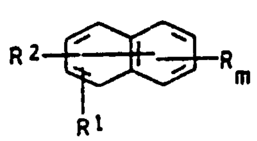

I

where R is $C_1$-$C_4$-alkyl, $C_1$-$C_4$-alkylthio, $C_1$-$C_4$-haloalkyl, $C_1$-$C_4$-alkoxy, halogen, hydroxyl, nitro or benzyl,
m is 0, 1, 2 or 3, and the radicals R may be different when m is 2 or 3,
$R^1$ is -CN, -C($NH_2$)NOH,

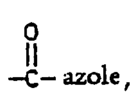 , 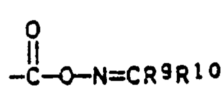 or 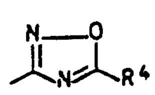 ;

$R^2$ is halogen, -$NR^5R^6$, N = CH-$R^5$, -$NR^5$-$SO_2R^7$, $COOR^3$, OH

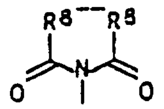 or 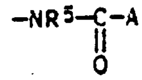

and

$R^3$ is hydrogen,

$C_1$-$C_6$-alkyl which may carry from one to three of the following substituents: halogen, $C_1$-$C_4$-alkylamino, di-($C_1$-$C_4$-alkyl)-amino, $C_1$-$C_4$-alkoxy or $C_1$-$C_4$-alkylthio, phenyl or benzyl which may carry from one to three of the following groups: $C_1$-$C_4$-alkyl, $C_1$-$C_4$-alkoxy, $C_1$-$C_4$-alkylthio, $C_1$-$C_4$-haloalkyl, cyano, nitro and/or halogen,

$R^4$ is phenyl which may carry one or two of the following groups: halogen, $C_1$-$C_4$-alkyl, $C_1$-$C_4$-alkoxy, $C_1$-$C_4$-alkylthio, $C_1$-$C_4$-haloalkyl, cyano and/or nitro,

$R^5$ is hydrogen or

$C_1$-$C_6$-alkyl which may carry from one to three of the following substituents: halogen, $C_1$-$C_4$-alkylamino, di-($C_1$-$C_4$-alkyl)-amino, $C_1$-$C_4$-alkoxy, $C_1$-$C_4$-alkylthio, phenyl and/or ($C_1$-$C_4$-alkyl)-oxycarbonyl, or

phenyl which may carry from one to three of the following substituents: halogen, $C_1$-$C_4$-alkyl, $C_1$-$C_4$-alkoxy, $C_1$-$C_4$-alkylthio, $C_1$-$C_4$-haloalkyl, cyano and/or nitro, $R^6$ is hydrogen or

$C_1$-$C_6$-alkyl which may carry from one to three of the following substituents: halogen, $C_1$-$C_4$-alkylamino, di-($C_1$-$C_4$-alkyl)-amino, $C_1$-$C_4$-alkoxy, $C_1$-$C_4$-alkylthio and/or phenyl,

$R^7$ is $C_1$-$C_6$-alkyl which may carry from one to three of the following substituents: $C_1$-$C_4$-alkoxy, $C_1$-$C_4$-alkylthio, $C_1$-$C_4$-alkylamino, di-($C_1$-$C_4$-alkyl)-amino, phenyl and/or from one to five halogen atoms, or

phenyl or 5-membered or 6-membered hetaryl, and these rings may carry from one to three of the following groups: $C_1$-$C_4$-alkyl, $C_1$-$C_4$-alkoxy, $C_1$-$C_4$-alkylthio, $C_1$-$C_4$-haloalkyl, nitro, cyano and/or halogen, $R^8$ is $C_1$-$C_6$-alkyl or $C_2$-$C_6$-alkenyl, and these groups may carry from one to three of the following substituents: halogen, $C_1$-$C_4$-alkoxy, $C_1$-$C_4$-alkylthio and/or phenyl, or

phenyl which may carry from one to three of the following substituents: $C_1$-$C_4$-alkyl, $C_1$-$C_4$-alkoxy, $C_1$-$C_4$-alkylthio, $C_1$-$C_4$-haloalkyl, nitro, cyano and/or halogen,

or $R^8$...$R^8$ together form a $C_2$-$C_4$-alkylene or $C_2$-$C_4$-alkenylene chain, and these bridges may carry up to three of the following substituents: $C_1$-$C_4$-alkyl, $C_1$-$C_4$-alkoxy, $C_1$-$C_4$-alkylthio, halogen and/or phenyl, or

may form a fused benzene ring which may carry from one to four halogen atoms and/or from one to three of the following radicals: $C_1$-$C_4$-aryl, $C_1$-$C_4$-alkoxy, $C_1$-$C_4$-alkylthio, $C_1$-$C_4$-haloalkyl, cyano, nitro and/or phenyl,

$R^9$ and $R^{10}$ are each $C_1$-$C_4$-alkyl or, together with the carbon atom to which they are bonded, are $C_3$-$C_6$-cycloalkyl, and

A is hydrogen,

$C_1$-$C_6$-alkyl which may carry from one to three of the following substituents: halogen, $C_1$-$C_4$-alkoxy, $C_1$-$C_4$-alkylthio, $C_1$-$C_4$-alkylamino, di-($C_1$-$C_4$-alkyl)-amino, phenyl and/or $C_1$-$C_4$-alkoxycarbonyl,

$C_1$-$C_4$-alkoxycarbonyl,

-$NR^5R^6$- or

phenyl which may carry from one to five halogen atoms and/or from one to three of the following substituents:

$C_1$-$C_4$-alkyl, $C_1$-$C_4$-alkoxy, $C_1$-$C_4$-haloalkyl, cyano and/or nitro,

and at least one herbicidal active ingredient from the group consisting of

a) the 2-(4-hetaryloxy)- or 2-(4-aryloxy)-phenoxyacetic acid derivatives of the formula II

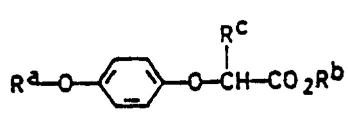 II

where $R^a$ is a phenyl ring, a pyridyl ring, a benzoazyl radical, a benzothiazyl radical or a benzopyrazinyl radical, and these aromatic ring systems may carry one or two of the following radicals: halogen, $C_1$-$C_4$-alkyl, $C_1$-$C_4$-haloalkyl and/or nitro,

$R^b$ is hydrogen, $C_1$-$C_4$-alkyl or one equivalent of a plant-tolerated cation and

$R^c$ is hydrogen or methyl, and/or

b) the cyclohexenone derivatives of the formula III

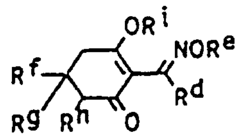

III

where

$R^d$ is $C_1$-$C_4$-alkyl,

$R^e$ is $C_1$-$C_4$-alkyl, $C_3$- or $C_4$-alkenyl, $C_3$- or $C_4$-alkynyl, $C_3$- or $C_4$-haloalkylene or thenyl which may be substituted by halogen,

$R^f$ is $C_1$-$C_4$-alkyl which may be monosubstituted by $C_1$-$C_4$-alkylthio or $C_1$-$C_4$-alkoxy,

a 5-membered or 6-membered saturated or monounsaturated ring system which, in addition to carbon members, may contain an oxygen or sulfur atom, and the sulfur atom may carry one or two oxygen atoms and this ring may carry up to three of the following radicals: hydroxyl, halogen, $C_1$-$C_4$-alkyl, $C_1$-$C_4$-haloalkyl, $C_1$-$C_4$-alkoxy and/or $C_1$-$C_4$-alkylthio,

a 10-membered saturated or monounsaturated heterocyclic structure which contains two oxygen or sulfur atoms and may be substituted by up to three $C_1$-$C_4$-alkyl groups and/or methoxy groups,

phenyl, pyridyl or isoxazolyl, and these radicals may carry up to three of the following groups: $C_1$-$C_4$-alkyl,$C_1$-$C_4$-haloalkyl, $C_1$-$C_4$-alkoxy, $C_1$-$C_4$-alkylthio, $C_3$-$C_6$-alkenyloxy, $C_3$-$C_6$-alkynyloxy, $C_1$-$C_4$-alkoxy-$C_1$-$C_3$-alkyl, $C_1$-$C_4$-dialkoxy-$C_1$-$C_3$-alkyl, formyl, halogen and/or benzoylamino

$R^g$ is hydrogen or $C_1$-$C_6$-alkyl,

$R^h$ is hydrogen, cyano, halogen or $C_1$-$C_4$-alkoxycarbonyl and

$R^i$ is hydrogen or one equivalent of an environmentally compatible cation.

**2.** A herbicide as claimed in claim 1, wherein the weight ratio of herbicide of the formula II to naphthalene derivative, applied together or separately, is from 1:4 to 1:0.01 parts.

**3.** A herbicide as claimed in claim 1, wherein the weight ratio of herbicide of the formula III to naphthalene derivative, applied together or separately, is from 1:4 to 1:0.01 parts.

**4.** A method for selectively controlling undesirable plant growth, wherein a naphthalene derivative of the formula I as claimed in claim 1 and a 2-(4-heteroaryloxy)- or 2-(4-aryloxy)-phenoxyacetic acid derivative of the formula II are applied, simultaneously or one after the other, before, during or after sowing of the crops, or before or during emergence of the crops.

**5.** A method for selectively controlling undesirable plant growth, wherein a naphthalene derivative of the formula I as claimed in claim 1 and a cyclohexenone derivative of the formula III are applied, simultaneously or one after the other in any order, before, during or after sowing of the crops, or before or during emergence of the crops.

**6.** A method for preventing damage to crops by herbicidal 2-(4-heteroaryloxy)- or 2(4-aryloxy)-phenoxyacetic acid derivatives of the formula II as claimed in claim 1, wherein the seed of the crops is treated with an antagonistic amount of a naphthalene derivative of the formula I as claimed in claim 1.

**7.** A method for preventing damage to crops by herbicidal cyclohexenone derivatives of the formula III as claimed in claim 1, wherein the seed of the crops is treated with antagonistic amount of a naphthalene derivative of the formula I as claimed in claim 1.

**8.** A method for selectively controlling undesirable plant growth, wherein the leaves of the crops and the undesirable plants are treated postemergence with a naphthalene derivative of the formula I as claimed in claim 1 and with a 2-(4-heteroaryloxy)- or 2-(4-aryloxy)-phenoxyacetic acid derivative of the formula II, either simultaneously or one after the other.

**9.** A method for selectively controlling undesirable plant growth, wherein the leaves of the crops and the undesirable plants are treated postemergence with a naphthalene derivative of the formula I as claimed in claim 1 and with a cyclohexenone derivative of the formula III, either simultaneously or one after the other.

**10.** A method as claimed in any of claims 4 to 9, wherein the crops are barley, wheat, corn, sorghum and rice.

## Revendications

**1.** Agents herbicides, caractérisés en ce qu'ils contiennent au moins un dérivé de naphtalène de formule I

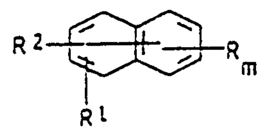

I

dans laquelle les substituants et l'indice ont la signification suivante:

| | |
|---|---|
| R | représente un groupe alkyle en $C_1$-$C_4$, alkylthio en $C_1$-$C_4$, halogénoalkyle en $C_1$-$C_4$, alcoxy en $C_1$-$C_4$, un halogène ou un groupe hydroxy, nitro ou benzyle; |
| m | vaut 0, 1, 2 ou 3, tandis que les restes R peuvent être différents lorsque m vaut 2 ou 3; |
| $R^1$ | est -CN; $-C(NH_2)NOH$; |

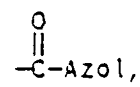, 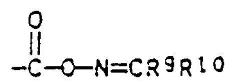 ou 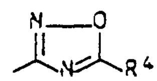 ;

| | |
|---|---|
| $R^2$ | est un halogène ou $-NR^5R^6$; $-N=CH-R^5$; $-NR^5-SO_2R^7$; $COOR^3$; OH |

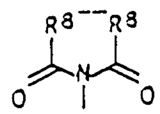 ou 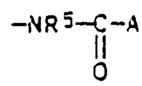

| | |
|---|---|
| | et |
| $R^3$ | est l'hydrogène ou un groupe alkyle en $C_1$-$C_6$, pouvant porter un à trois des substituants suivants: halogène, alkylamino en $C_1$-$C_4$, di-(alkyl en $C_1$-$C_4$)amino, alcoxy en $C_1$-$C_4$ et alkylthio en $C_1$-$C_4$; phényle ou benzyle, pouvant porter un à trois des groupes suivants: alkyle en $C_1$-$C_4$, alcoxy en $C_1$-$C_4$, alkylthio en $C_1$-$C_4$, halogénoalkyle en $C_1$-$C_4$, cyano, nitro et/ou halogène; |
| $R^4$ | est un groupe phényle, pouvant porter un ou deux des groupes suivants: halogène, alkyle en $C_1$-$C_4$, alcoxy en $C_1$-$C_4$, alkylthio en $C_1$-$C_4$, halogénoalkyle en $C_1$-$C_4$, cyano et/ou nitro; |
| $R^5$ | est l'hydrogène ou un groupe alkyle en $C_1$-$C_6$, pouvant porter un à trois des substituants suivants: halogène, alkylamino en $C_1$-$C_4$, di-(alkyl en $C_1$-$C_4$)amino, alcoxy en $C_1$-$C_4$, alkylthio en $C_1$-$C_4$, phényle et/ou (alkyl en $C_1$-$C_4$)oxycarbonyle; phényle, pouvant porter un à trois des substituants suivants: halogène, alkyle en $C_1$-$C_4$, alcoxy en $C_1$-$C_4$, alkylthio en $C_1$-$C_4$, halogénoalkyle en $C_1$-$C_4$, cyano et/ou nitro; |
| $R^6$ | est l'hydrogène ou un groupe alkyle en $C_1$-$C_6$, pouvant porter un à trois des substituants suivants: halogène, alkylamino en $C_1$-$C_4$, di-(alkyl en $C_1$-$C_4$)amino, alcoxy en $C_1$-$C_4$, alkylthio en $C_1$-$C_4$ et/ou phényle; |
| $R^7$ | est un groupe |

alkyle en $C_1$-$C_6$, pouvant porter un à trois des substituants suivants: alcoxy en $C_1$-$C_4$, alkylthio en $C_1$-$C_4$, alkylamino en $C_1$-$C_4$, di-(alkyl en $C_1$-$C_4$)amino, phényle et/ou un à cinq atomes d'halogène;

phényle ou hétéroaryle à 5 à 6 chaînons, ces cycles pouvant porter un à trois des groupes suivants: alkyle en $C_1$-$C_4$, alcoxy en $C_1$-$C_4$, alkylthio en $C_1$-$C_4$, halogénoalkyle en $C_1$-$C_4$, nitro, cyano et/ou halogène;

$R^8$ est un groupe

alkyle en $C_1$-$C_6$ ou alcényle en $C_2$-$C_6$, ces groupes pouvant porter un à trois des substituants suivants: halogène, alcoxy en $C_1$-$C_4$, alkylthio en $C_1$-$C_4$ et/ou phényle;

phényle, pouvant porter un à trois des substituants suivants: alkyle en $C_1$-$C_4$, alcoxy en $C_1$-$C_4$, alkylthio en $C_1$-$C_4$, halogénoalkyle en $C_1$-$C_4$, nitro, cyano et/ou halogène;

ou $R^8$...$R^8$ représentent ensemble

une chaîne alkylène en $C_2$-$C_4$ ou alcényle en $C_2$-$C_4$, ces ponts pouvant porter jusqu'à trois des substituants suivants: alkyle en $C_1$-$C_4$, alcoxy en $C_1$-$C_4$, alkylthio en $C_1$-$C_4$, halogène et/ou phényle, ou

un cycle benzénique condensé, pouvant porter un à quatre atomes d'halogène et/ou un à trois des restes suivants: alkyle en $C_1$-$C_4$, alcoxy en $C_1$-$C_4$, alkylthio en $C_1$-$C_4$, halogénoalkyle en $C_1$-$C_4$, cyano, nitro et/ou phényle;

$R^9$, $R^{10}$ représentent un groupe alkyle en $C_1$-$C_4$ ou, conjointement avec l'atome de carbone auquel ils sont liés, un groupe cycloalkyle en $C_3$-$C_6$;

A représente l'hydrogène ou un groupe

alkyle en $C_1$-$C_6$, pouvant porter un à trois des substituants suivants: halogène, alcoxy en $C_1$-$C_4$, alkylthio en $C_1$-$C_4$, alkylamino en $C_1$-$C_4$, di-(alkyl en $C_1$-$C_4$)amino, phényle et/ou (alcoxy en $C_1$-$C_4$)carbonyle;

(alcoxy en $C_1$-$C_4$)carbonyle;

$-NR^5R^6-$ ou

phényle, pouvant porter un à cinq atomes d'halogène et/ou un à trois des substituants suivants: alkyle en $C_1$-$C_4$, alcoxy en $C_1$-$C_4$, halogénoalkyle en $C_1$-$C_4$, cyano et/ou nitro;

comme agent antagoniste, ainsi qu'au moins une substance à activité herbicide choisie dans le groupe

a) des dérivés d'acide 2-(4-hétéroaryloxy)- ou 2-(4-aryloxy)-phénoxyacétique de formule II

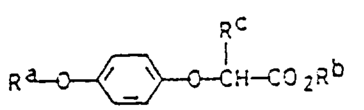

II

dans laquelle les substituants ont les significations suivantes:

$R^a$ représente un noyau phényle, un cycle pyridine, un reste benzoxazyle, un reste benzothiazyle ou un reste benzopyrazinyle, ces systèmes cycliques aromatiques pouvant porter un à deux des restes suivants: halogène, alkyle en $C_1$-$C_4$, halogénoalkyle en $C_1$-$C_4$, et/ou nitro;

$R^b$ représente l'hydrogène,

un groupe alkyle en $C_1$-$C_4$ ou l'équivalent d'un cation compatible avec les plantes et

$R^c$ l'hydrogène ou un groupe méthyle et/ou,

b) les dérivés de cyclohexénone de formule III

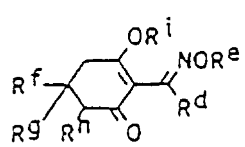

III

dans laquelle les substituants ont la signification suivante:

$R^d$ un groupe alkyle en $C_1$-$C_4$;

$R^e$ un groupe alkyle en $C_1$-$C_4$, un groupe alcényle en $C_3$-$C_4$, un groupe alcynyle en $C_3$-$C_4$, un groupe halogénoalkylène en $C_3$-$C_4$ ou un groupe thiényle, pouvant être substitué par un atome d'halogène;

$R^f$ un groupe alkyle en $C_1$-$C_4$, pouvant être substitué une fois par un groupe alkylthio en $C_1$-$C_4$ ou alcoxy en $C_1$-$C_4$;
un système cyclique à 5 ou 6 chaînons, saturé ou une fois insaturé, pouvant porter, en plus des chaînons carbonés, un atome d'oxygène ou de soufre, tandis que l'atome de soufre peut porter jusqu'à deux atomes d'oxygène, et ce cycle pouvant porter jusqu'à trois des restes suivants: hydroxy, halogène, alkyle en $C_1$-$C_4$, halogénoalkyle en $C_1$-$C_4$, alcoxy en $C_1$-$C_4$ et/ou alkylthio en $C_1$-$C_4$;
un hétérocycle à 10 chaînons, saturé ou une fois insaturé, contenant deux atomes d'oxygène ou atomes de soufre et pouvant être substitué par jusqu'à trois groupes alkyle en $C_1$-$C_4$ et/ou groupes méthoxy;
un reste phényle, un reste pyridyle ou un reste isoxazolyle, ces restes pouvant porter jusqu'à trois des groupes suivants: alkyle en $C_1$-$C_4$, halogénoalkyle en $C_1$-$C_4$, alcoxy en $C_1$-$C_4$, alkylthio en $C_1$-$C_4$, alcényloxy en $C_3$-$C_6$, alcynyloxy en $C_3$-$C_6$, (alcoxy en $C_1$-$C_4$)-alkyle en $C_1$-$C_3$, di(alcoxy en $C_1$-$C_4$)alkyle en $C_1$-$C_3$, formyle, halogène et/ou benzoylamino;

$R^g$ l'hydrogène ou un groupe alkyle en $C_1$-$C_6$;

$R^h$ l'hydrogène, un groupe cyano, un atome d'halogène ou un groupe (alcoxy en $C_1$-$C_4$)-carbonyle et

$R^i$ l'hydrogène ou l'équivalent d'un cation acceptable pour l'environnement.

2. Agent herbicide selon la revendication 1, caractérisé en ce que le rapport de fractions d'herbicide de formule II/dérivé de naphtalène s'élève pour une application simultanée ou séparée à 1:4 à 1:0,01 parties en poids.

3. Agent herbicide selon la revendication 1, caractérisé en ce que le rapport de fractions d'herbicide de formule III/dérivé de naphtalène s'élève pour une application simultanée ou séparée à 1:4 à 1:0,01 parties en poids.

4. Procédé pour la lutte sélective contre la croissance des plantes parasites, caractérisé en ce qu'on applique simultanément ou séparément un dérivé de naphtalène de formule I selon la revendication 1 et un dérivé d'acide 2-(4-hétéroaryloxy)- ou 2-(4-aryloxy)-phénoxyacétique de formule II avant, pendant ou après le semis des plantes cultivées, avant ou pendant la levée des plantes cultivées.

5. Procédé pour la lutte sélective contre la croissance des plantes parasites, caractérisé en ce qu'on applique simultanément ou l'un après l'autre dans un ordre quelconque un dérivé de naphtalène de formule I selon la revendication 1 et un dérivé de cyclohexénone de formule III avant, pendant ou après le semis des plantes cultivées, avant ou pendant la levée des plantes cultivées.

6. Procédé pour empêcher les dommages aux plantes cultivées au moyen de dérivés herbicides d'acide 2-(4-hétéroaryloxy)- ou 2-(4-aryloxy)-phénoxyacétique de formule II selon la revendication 1, caractérisé en ce qu'on traite les semences des plantes cultivées avec une quantité à activité antagoniste d'un dérivé de naphtalène de formule I selon la revendication 1.

7. Procédé pour empêcher les dommages aux plantes cultivées au moyen de dérivés herbicides de cyclohexénone de formule III selon la revendication 1, caractérisé en ce qu'on traite les semences des plantes cultivées avec une quantité à activité antagoniste d'un dérivé de naphtalène de formule I selon la revendication 1.

8. Procédé pour lutter sélectivement contre la croissance des plantes parasites, caractérisé en ce qu'on traite les feuilles des plantes cultivées et des plantes parasites dans le procédé en post-levée, avec un dérivé de naphtalène de formule I selon la revendication 1 et avec un dérivé d'acide 2-(4-hétéroaryloxy)- ou 2-(4-aryloxy)-phénoxyacétique de formule II, simultanément ou successivement.

9. Procédé pour lutter sélectivement contre la croissance des plantes parasites, caractérisé en ce qu'on traite les feuilles des plantes cultivées et des plantes parasites dans le procédé en post-levée, avec un dérivé de naphtalène de formule I selon la revendication 1 et avec un dérivé de cyclohexénone de formule III, simultanément ou successivement.

10. Procédé selon l'une quelconque des revendications 4 à 9, caractérisé en ce que les plantes cultivées sont l'orge, le blé, le maïs, le sorgho cultivé et le riz.